Europäisches Patentamt

European Patent Office

Office européen des brevets

(19)

(11) Veröffentlichungsnummer : **0 380 989 B1**

(12) **EUROPÄISCHE PATENTSCHRIFT**

(45) Veröffentlichungstag der Patentschrift :
23.12.92 Patentblatt 92/52

(21) Anmeldenummer : 90101156.9

(22) Anmeldetag : 20.01.90

(51) Int. Cl.⁵ : $A61K\ 47/18$, $A61L\ 15/16$

(30) Priorität : 25.01.89 DE 3902013

(43) Veröffentlichungstag der Anmeldung :
08.08.90 Patentblatt 90/32

(45) Bekanntmachung des Hinweises auf die
Patenterteilung :
23.12.92 Patentblatt 92/52

(84) Benannte Vertragsstaaten :
AT BE CH DE FR GB IT LI NL SE

(56) Entgegenhaltungen :
DE-A- 3 131 071

(54) Pflaster zur transdermalen Anwendung.

(56) Entgegenhaltungen :
STN INTERNATIONAL INFORMATION SERVI-
CES, DATENBANK, CHEMICAL ABSTRACTS,
Band 102, Nr. 12, Zusammenfassung Nr.
100739r,Columbus, Ohio, US; H.D. TAUSCHEL
et al.: "Studies on the percutaneous activity of
a heparin-allantoin-dexpanthenol combination ina specific ointment base. Thrombolytic
activity on the rabbit ear", & ARNZNEIM.-
FORSCH., 34(12), 1768-72, 1984

(73) Patentinhaber : KNOLL AG
Knollstrasse 32
W-6700 Ludwigshafen (DE)

(72) Erfinder : Kolter, Karl, Dr.
Sachsenweg 12
W-6703 Limburgerhof (DE)
Erfinder : Rieker, Aribert, Dr.
Sternstrasse 63
W-6700 Ludwigshafen (DE)

(74) Vertreter : Michaelis, Wolfgang, Dr. et al
c/o BASF Aktiengesellschaft
Carl-Bosch-Strasse 38
W-6700 Ludwigshafen (DE)

EP 0 380 989 B1

## Beschreibung

Die Erfindung betrifft ein Pflaster zur transdermalen Anwendung systemisch wirksamer pharmazeutischer Wirkstoffe mit Dexpanthenol als Penetrationshilfsmittel.

Dexpanthenol wird - allein oder zusammen mit anderen Wirkstoffen - in Salben als Heilmittel für Verletzungen und Entzündungen der Haut verwendet.

Beim Einsatz von Pflastern zur transdermalen Anwendung systemisch wirksamer pharmazeutischer Wirkstoffe, wobei die Wirkstoffe durch die Haut in den Blutkreislauf gelangen sollen, ist für viele Wirkstoffe die Passage durch die Haut problematisch. Daher werden häufig Penetrationsbeschleuniger eingesetzt. Als solche sind beispielsweise bekannt: Dimethylsulfoxid, Azone (1-Dodecylhexanydro-2H-azepin-2-on), Dimethylacetamid, N-Methyl-2-pyrrolidon, Ölsäure.

Sie haben jedoch folgende Nachteile: Auslösung von Hautirritationen, Hautentzündungen, Verstärkung von hautirritierenden Wirkungen von Arzneistoffen, toxikologisch nicht völlig unbedenklich.

Der Erfindung lag die Aufgabe zugrunde, ein Penetrationshilfsmittel zu finden, das von den oben genannten Nachteilen frei ist.

Es wurde gefunden, daß Dexpanthenol hervorragend als Penetrationshilfsmittel in Pflastern für systemisch wirksame Pharmaka mit Molekulargewichten unter 1000 geeignet ist und hautirritierende Einflüsse von Wirk- und Hilfsstoffen unterdrückt oder vermindert.

In CA-102 (12) Ref. Nr. 100739 r wird für die Kombination von Allantoin und Dexpanthenol eine penetrationsbeschleunigende Wirkung auf Heparin beschrieben. Heparin ist jedoch ein Polymeres vom Molekulargewicht etwa 17000 und hat mit den niedermolekularen Wirkstoffen, auf die sich die Erfindung bezieht, nichts gemein.

Das Pflaster kann in beliebiger Weise aufgebaut sein, beispielsweise nach dem Matrixsystem, dem Membransystem oder dem Vliessystem (Drug Dev. Ind. Pharm. 14 (1988), 183-209; Drug Dev. Ind. Pharm. 13 (1987), 589-651; Drugs of Today 23 (1987), 625-646.

Das Matrixsystem besteht in einfachster Weise aus 3 Teilen: der flexiblen Stützfolie, der den Wirkstoff enthaltenden Klebematrix und einer Abziehfolie. Falls eine nichtklebende Matrix verwendet wird, muß zur Haftung auf der Haut eine Randzone der Stützfolie mit Klebstoff versehen werden.

Ein Membransystem weist hingegen mindestens 5 Teile auf: eine flexible Stützfolie, ein Reservoir mit gelöstem oder suspendiertem Wirkstoff, eine Membran zur Steuerung der Wirkstofffreigabe, eine auf die Membran aufgezogene Klebeschicht und eine Abziehfolie.

Beim Vliessystem besteht die den Wirkstoff enthaltende Schicht aus einem saugfähigen Vlies oder porösen Polymer, das mit einer Wirkstofflösung oder -suspension getränkt ist. Diese Schicht, die fest mit der Stützfolie verbunden ist, wird durch eine Abziehfolie abgedeckt. Der Rand der Stützfolie ist, zur Applikation auf die Haut, mit Klebstoff versehen.

Grundsätzlich sind alle systemisch wirksamen Pharmaka mit Molekulargewichten unter 1000 erfindungsgemäß einsetzbar. Bevorzugt werden Analgetika, insbesondere Opiate, Calcium-Antagonisten, Antihypertensiva, Antiarrhythmika, Beta-Rezeptorenblocker, Psychopharmaka, Vasodilatatoren, Antiparkinson-Mittel, Anticholinergika, Antihistaminika, Antirheumatika und Hormone. Besonders bevorzugt werden die Wirkstoffe Hydromorphon, Biperiden, Gallopamil und Soquinolol.

Die zu verwendenden Hilfsstoffe sind die für die Herstellung von Pflastern üblichen. Neben dem klebenden Agens, in der Regel ein Polymer mit einer Glastemperatur zwischen -70 und -10, vorzugsweise -55 und -25°C, sowie einer Trägerfolie, die mit diesem klebenden Agens beschichtet wird, dem Wirkstoff und dem Dexpanthenol werden häufig Emulgatoren, Verdickungsmittel sowie Stoffe, die die Wirkstofffreigabe beeinflussen sollen, und andere Hilfsmittel zugesetzt.

Die klebrigen Polymeren mit den oben genannten niedrigen Glastemperaturen sind bekannt, beispielsweise aus den US-Patenten 2 973 282 und 3 307 544. Die selbstklebenden Bänder und Folien sollen auf bloßen Kontakt auf der menschlichen Haut kleben, doch soll die Kohäsion der Klebschicht und deren Adhäsion an der Trägerfolie größer sein als die Adhäsion an der Haut, so daß sie sich weitgehend rückstandslos wieder abziehen lassen. Es handelt sich in der Regel um Copolymerisate auf der Basis von Acryl- und Methacrylsäureestern von Alkoholen mit 2 bis 12, vorzugsweise 4 bis 8 Kohlenstoffatomen, die zahlreiche andere Comonomere einpolymerisiert enthalten können, beispielsweise (Meth)acrylsäure, (Meth)acrylnitril, (Meth)acrylamid, N-tert.-Butyl-(meth-)acrylamid, Vinylester wie Vinylacetat, -propionat oder -butyrat, andere Vinylverbindungen wie Styrol, ferner Butadien. Besonders hervorgehoben seien Butylacrylat und 2-Ethylhexylacrylat. Die Polymeren können durch Zusatz geringer Mengen Comonomerer mit 2 oder mehr copolymerisierbaren Doppelbindungen, also beispielsweise von Diacrylaten, wie Butandioldiacrylat, oder Divinylverbindungen, wie Divinylbenzol, oder durch Zusatz anderer Vernetzungsmittel, z.B. Melamin-Formaldehydharze, vernetzt sein. Als klebrige Polymere können ferner Polyisobutylene und Polyvinylether unterschiedlichen Molekulargewichts verwendet werden.

Die Teilchengröße der Dispersionen soll zwischen 50 und 500 nm, bevorzugt zwischen 50 und 200 nm liegen. Die Teilchengröße und der Vernetzungsgrad können in bekannter Weise in Abhängigkeit von den Polymerisationsbedingungen und den Comonomeren eingestellt werden. Kleinere Teilchengrößen und ein erhöhter Vernetzungsgrad können eine Steigerung der Wirkstofffreisetzung bewirken.

Die erfindungsgemäßen Matrix-Pflaster können in üblicher Weise hergestellt werden, indem man den Wirkstoff und das Dexpanthenol in einer geeigneten Polymerlösung löst oder fein dispergiert und anschließend diese wirkstoffhaltige Selbstklebemasse mittels Walzen- oder Rakelauftragsverfahren zum Film auszieht. In einigen Fällen ist es zweckmäßig, den Wirkstoff oder das Dexpanthenol oder beide vor der Zugabe zu der Polymerlösung in einem organischen Lösungsmittel, z.B. Ethanol oder Aceton, aufzulösen oder feinst zu dispergieren. Dadurch kann eine bessere Verteilung des Wirkstoffes und des Dexpanthenols im Polymer erzielt werden.

Die erfindungsgemäßen Pflaster können auch nach der deutschen Patentanmeldung P 38 07 283.1 hergestellt werden, indem man den Wirkstoff in feinpulvriger Form (Teilchengröße unter 200, vorzugsweise unter 50 μm) und das Dexpanthenol gemeinsam oder einzeln in die wäßrige Latexdisperison einarbeitet, oder indem man beide in einer wäßrigen Emulgatorlösung dispergiert oder löst und diese Mischung der wäßrigen Latexdispersion bei einer Temperatur von 10 bis 80, bevorzugt 30 bis 70°C zumischt. Daneben kann auch das Salz eines sauren oder basischen Wirkstoffs in wäßriger Lösung mit der Polymerdispersion bei einem pH-Wert gemischt werden, bei dem der Wirkstoff vorwiegend in der wasserlöslichen ionisierten Form vorliegt. Durch pH-Verschiebung wird dann der Wirkstoff in die ungeladene wasserunlösliche Form gebracht und simultan in die Dispersion einemulgiert.

Zweckmäßig legt man den Wirkstoff und das Dexpanthenol vor, gibt den Emulgator und Wasser zu und mischt dann mit der Polymerdispersion. Die so erhaltene wirkstoffhaltige Dispersion wird gegebenenfalls mit weiteren Hilfsstoffen versehen und, wie erwähnt, in an sich bekannter Weise auf einer Stützfolie zu einem Film ausgezogen und getrocknet. Die Trocknungstemperatur kann hierbei zwischen Raumtemperatur und 100°C liegen, wobei ein Optimum zwischen angestrebter schneller Trocknung und zu vermeidender Blasenbildung im Film sowie thermischer Belastung des Wirkstoffs im allgemeinen bei 35 bis 45°C liegt.

Dieses Verfahren hat den großen Vorteil der Vermeidung von organischen Lösungsmitteln. Jedoch kommen im Prinzip auch alle anderen üblichen Herstellverfahren für Matrix-Pflaster in Betracht.

Die resultierenden Filme haben Dicken von 10 bis 800, bevorzugt 50 bis 300 μm. Die Filmherstellung kann kontinuierlich oder diskontinuierlich erfolgen. Das Auftrageverfahren kann mehrmals wiederholt werden, bis der Film die gewünschte Dicke erreicht hat. Die klebrige Polymerschicht enthält den Wirkstoff in einer Konzentration im Bereich von 1 bis 40, vorzugsweise 5 bis 25 Gew.%, Dexpanthenol in einer Konzentration im Bereich von 0,1 bis 50, vorzugsweise 1 bis 20 Gew.%. Die gleiche Dexpanthenolkonzentration gilt auch für die Reservoirflüssigkeit beim Membransystem und für die Wirkstofflösung oder -Dispersion, mit der beim Vliessystem das Vlies oder das poröse Polymere getränkt wird.

Als Emulgatoren sowohl für die Wirkstoffe wie auch die Polymeren werden die hierfür üblichen Tenside verwendet, wie das Natriumsalz von längerkettigen Fettsäuren und des Schwefelsäurehalbesters eines (gegebenenfalls oxethylierten) Fettalkohols als Beispiele für anionische Tenside sowie polyoxethylierte Alkylphenole und längerkettige Fettalkohole (z.B. Hexadecan-(1)-ol) und Glycerin-Fettsäurepartialester als Beispiele für nichtionische Tenside und Coemulgatoren.

Die gewünschte Viskosität der ausziehfertigen Masse kann z.B. mit Polyacrylsäuren oder Cellulosederivaten eingestellt werden.

Als zusätzliche Vernetzungsmittel die die Kohäsion und damit die Klebeeigenschaften der Filme verbessern, können z.B. Melamin-Formaldehydharze verwendet werden.

Im Sinne einer Verbeserung der Wirkstofffreisetzung wirken Quellstoffe wie Polyvinylpyrrolidon, Cellulosederivate oder Polyacrylate, da der Film vermehrt Wasser aufnehmen kann und dadurch der Diffusionswiderstand sinkt. Die Freisetzung der Wirkstoffe kann ferner verbessert werden durch den Zusatz von hydrophilen Weichmachern wie Glycerin, 1, 2-Propandiol der Polyethylenglykolen und lipophilen Weichmachern wie Triacetin, Dibutylphthalat oder Isopropylmyristat.

Matrixpflaster ergeben üblicherweise eine Wirkstofffreisetzung 1. Ordnung. Durch die Verwendung von Füllstoffen, die den Wirkstoff adsorbieren, wie Aerosil, mikrokristalline Cellulose oder Lactose, resultiert annähernd eine Freisetzung 0. Ordnung.

Die Stützfolie, auf die die wirkstoffhaltige Selbstklebemasse aufgetrocknet wird, ist zweckmäßig sowohl für den Wirkstoff wie für Wasserdampf praktisch undurchlässig. Sie kann z.B. aus einer Aluminium-Kunststoff-Verbundfolie, einer metallisierten Kunststofolie, einer Kunststofolie, die zur Wirkstoffseite hin mit einer Sperrschicht aus z.B. Polyvinylidenchlorid versehen ist, oder aus einer einfachen Kunststofolie, z.B. Polyesterfolie, bestehen.

Die erfindungsgemäßen Pflaster, die nach dem Membransystem aufgebaut sind, werden ebenfalls in üblicher Weise hergestellt (z.B. EP 0 186 071 A2, US 4 262 003), wobei das Dexpanthenol in der Reservoirflüssigkeit gelöst oder fein dispergiert wird. In besonderen Fällen kann auch die mikroporöse Membran mit einer dexpanthenolhaltigen Lösung getränkt oder das Dexpanthenol in die auf der mikroporösen Membran befindliche Klebstoffschicht eingearbeitet werden.

Die Herstellung der nach dem Vliessystem aufgebauten Pflaster erfolgt dadurch, daß auf der Stützfolie befestigte Vliese oder poröse Polymere mit einer Lösung oder Dispersion des Wirkstoffes in einem hydrophilen oder lipophilen Lösungsmittel oder Lösungsmittelgemisch, das Dexpanthenol gelöst oder dispergiert enthält, getränkt werden. Anschließend wird die undurchlässige Abziehfolie aufgebracht.

Die in den Beispielen genannten Teile und Prozente beziehen sich auf das Gewicht.

## Beispiel 1

18 Teile Dexpanthenol wurden mit 18 Teilen Ethanol und 54 Teilen Ethylcaprylat versetzt. In dieser Mischung wurden 10 Teile Hydromorphon unter starkem Rühren suspendiert. Mittels einer Dosierpumpe wurden jeweils 425 mg dieser Suspension entsprechend 42,5 mg Hydromorphon auf das Vlies eines vorgelegten Leerpflasters so aufgebracht, daß die Suspension homogen über dem Vlies verteilt war. Dieses Pflaster wurde anschließend mit einer Abziehfolie aus silikonisiertem Aluminium (30 µm Dicke) verschlossen.

Das Leerpflaster bestand aus einer kreisrunden, außen hautfarben lackierten Stützfolie aus Polyethylen/Aluminium/Polyamid mit einer Dicke von 60 µm und einem Durchmesser von 7,0 cm und dem zentrisch aufgebrachten Vlies (T 1556, Fa. Freudenberg, D-Weinheim) mit einem Durchmesser von 3,2 cm. Der Rand der Stützfolie war mit einem handelsüblichen Polyacrylatkleber versehen.

## Beispiel 2

Eine 5,5 x 6,5 cm große Aluminium-Polyethylenfolie (Tscheulin GmbH, D-Teningen) wurde gemuldet. Mittels einer Dosierpumpe wurden 1,00 g einer Lösung aus 10 Teilen Gallopamil, 18 Teilen Dexpanthenol und 72 Teilen Ethanol auf die Folie aufgebracht. In einem separaten Arbeitsgang erfolgte die Beschichtung einer mikroporösen Polypropylenfolie (Celgard® 2400, Celanese) mit einem handelsüblichen Acrylatkleber, der anschließend mit einer silikonisierten Polyesterfolie (Scotch Pak® 75µm, 3M) als Abziehfolie abgedeckt wurde. Dieses dreischichtige Foliensystem wurde nun auf die mit der Wirkstofflösung versehene Stützfolie so aufgebracht, daß sich die mikroporöse Membran innen befand. Nach Abdichtung des Wirkstoffreservoirs durch Heißsiegelung lag das fertige Wirkstoffpflaster vor.

## Beispiel 3

25 Teile Dexpanthenol und 25 Teile Gallopamil wurden in 100 Teilen einer 10 %igen Natriumlaurylsulfatlösung unter starkem Rühren und Erwärmen auf 50°C dispergiert. In die entstandene Emulsion wurden 880 Teile einer 50 %igen Butylacrylatdispersion eingerührt und die erhaltene wirkstoffhaltige Polymerdispersion mittels einer geeigneten Ausstreichrakel auf einer Polyesterfolie mit 15 µm Dicke (Fa. Kalle, D-Wiesbaden) ausgestrichen und bei 35 bis 40°C unter kontrollierter Luftfeuchtigkeit getrocknet. Je nach Rakeleinstellung resultierten Flächengewichte von 5 mg/cm², die durch Mehrfachauftrag weiter erhöht werden konnten. Der so hergestellte selbstklebende Film mit einem Wirkstoffgehalt von 5 % wurde mit einer silikonisierten Abziehfolie aus Polyester (Scotch Pak 75 µm, 3M) versehen und in die gewünschten Abmessungen geschnitten.

## Beispiel 4

In einer Mischung aus 10 Teilen Dexpanthenol und 40 Teilen Ethanol wurden 12,5 Teile Biperiden (Korngröße: 100 % < 50 µm) unter starkem Rühren suspendiert. In dieser Suspension wurden wiederum unter starkem Rühren 225 Teile einer 30 %igen Lösung eines Copolymeren aus Acrylsäureethylhexylester und Acrylsäure in Benzin gegeben und anschließend die Mischung mittels einer geeigneten Rakel auf einer 15 µm dicken aluminisierten Polyesterfolie (Fa. Hueck, D-Weiden) ausgestrichen. Die Trocknung erfolgte bei 35 bis 40°C. Je nach Rakeleinstellung resultierten Flächengewichte von 8 mg/cm², die durch Mehrfachauftrag weiter gesteigert werden konnten. Der so hergestellte selbstklebende Film wurde anschließend in üblicher Weise konfektioniert.

Wirkungen von Dexpanthenol und Eigenschaften der Pflaster

Permeation durch excidierte Rattenhaut

Zur Messung der Permeation der Wirkstoffe durch Rattenhaut (Bauchhaut von Sprague Dawley Ratten 200 bis 250 g, männlich) wurde die excidierte Haut horizontal in eine Durchflußzelle eingespannt. Dabei wurde darauf geachtet, daß die Haut hydrostatisch äquilibriert war. Die für die Permeation zur Verfügung stehende Hautfläche betrug 20 cm². Das Akzeptormedium (250 ml, 32°C ± 0,5°C) wurde mit einer Geschwindigkeit von 830 ml/h umgepumpt. Zu verschiedenen Zeitpunkten wurden Proben entnommen und auf ihren Wirkstoffgehalt untersucht. Das Volumen der Proben wurde durch neues Akzeptormedium ersetzt.

1 g einer Suspension aus 10 Teilen Hydromorphon, 18 Teilen Dexpanthenol, 18 Teilen Ethanol und 54 Teilen Ethylacrylat (Zusammensetzung entsprechend dem Beispiel 1) wurden mittels einer Pipette auf die Rattenhaut aufgebracht und die Permeation bestimmt. Als Akzeptormedium fungierte isotonischer Phosphatpuffer pH 7,0, der die Einhaltung von sink-Bedingungen gewährleistete.

In einem 2. Versuch wurde 1 g einer Lösung aus 10 Teilen Hydromorphon, 22,5 Teilen Ethanol und 67,5 Teilen Ethylcaprylat (Verhältnis Ethanol:Ethylcaprylat wie oben 2:6) mittels einer Pipette auf die Rattenhaut appliziert und die Permeation bestimmt.

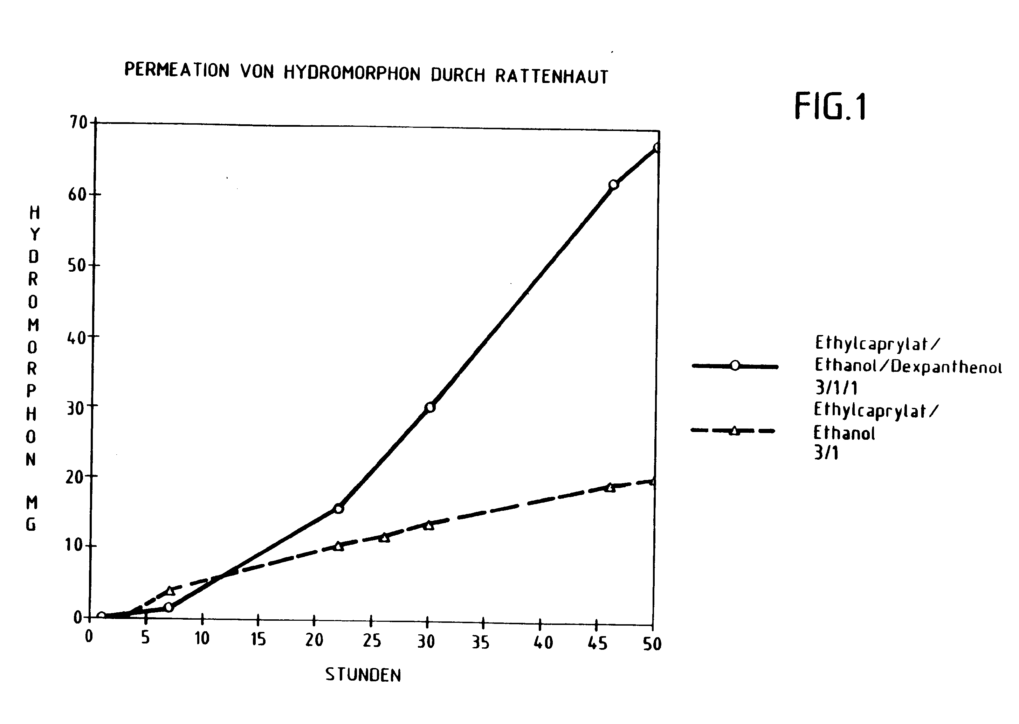

Die in Fig. 1 graphisch wiedergegebenen Permeationswerte belegen die permeationsbeschleunigende Wirkung von Dexpanthenol: Aus der Lösung mit Dexpanthenol traten nach 50 h 67,7 mg Hydromorphon durch die Haut, während aus der Lösung ohne Dexpanthenol nach 50 h nur 20,6 mg permeierten. Der Sättigungsflux betrug im ersten Fall 70,0 $\mu g/h/cm^2$ und im zweiten 13,4 $\mu/h/cm^2$. Somit ergibt sich für die permeationsbeschleunigende Wirkung des Dexpanthenols ein Faktor von 5,2.

Eine weitere Erhöhung des Dexpanthenolanteils in der Mischung auf 30 % ergab eine weitere Steigerung der Permeation auf 71,8 mg nach 50 h.

1 g einer Lösung aus 10 Teilen Gallopamil-Base, 18 Teilen Dexpanthenol und 72 Teilen Ethanol (Zusammensetzung analog Beispiel 2) wurden zur Permeationsmessung auf 20 cm² excidierte Rattenhaut appliziert. Als Akzeptormedium wurde isotonischer Phosphatpuffer pH 6,0 verwendet, der die Einhaltung von sink-Bedingungen über die gesamte Versuchsdauer ermöglichte.

Die Vergleichslösung ohne Dexpanthenol bestand aus einer 10 %igen Gallopamillösung in Ethanol.

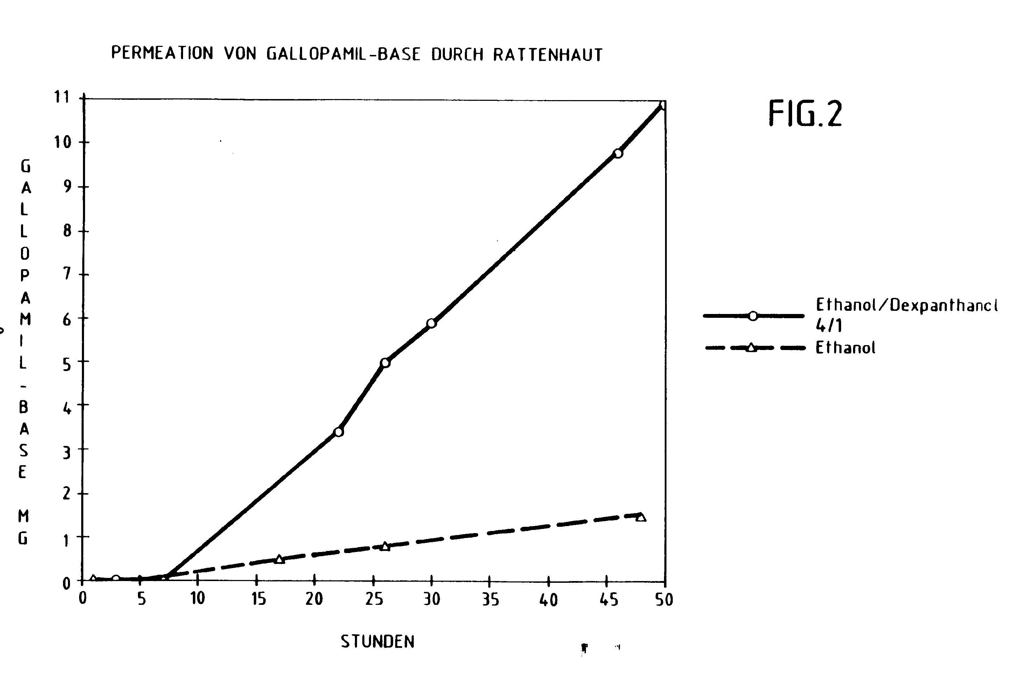

Fig. 2 zeigt, daß Dexpanthenol die Permeation von Gallopamil durch die Haut beschleunigt: Ohne Dexpanthenol wurden 1,5 mg Gallopamil nach 48 h, mit Dexpanthenol 10,9 mg Gallopamil nach 50 h im Akzeptormedium aufgefunden. Der Sättigungsflux betrug mit Dexpanthenol 12,7 $\mu g/h/cm^2$, ohne Dexpanthenol 1,8 $\mu g/h/cm^2$, wodurch sich ein Steigerungsfaktor von 7,1 ergibt.

Zur Bestimmung der Freisetzung des Wirkstoffes wurden Pflaster geeigneter Größe in eine Durchflußzelle eingespannt. Die für die Freigabe zur Verfügung stehende Pflasterfläche betrug 12,57 cm². Das Akzeptormedium (250 ml, 32°C ± 0,5°C) wurde mit einer Geschwindigkeit von 830 ml/h umgepumpt. Zu verschiedenen Zeitpunkten wurden Proben gezogen und auf ihren Wirkstoffgehalt untersucht. Das Volumen der Proben wurde durch neues Akzeptormedium ersetzt.

Substanz: Biperiden
Gehalt: 0,75 mg/cm² Biperiden
Filmgewicht: 8,0 mg/cm²
Herstellungsverfahren: analog Beispiel 4, mit und ohne Dexpanthenol
Akzeptormedium: isotonischer Phosphatpuffer pH 6,0

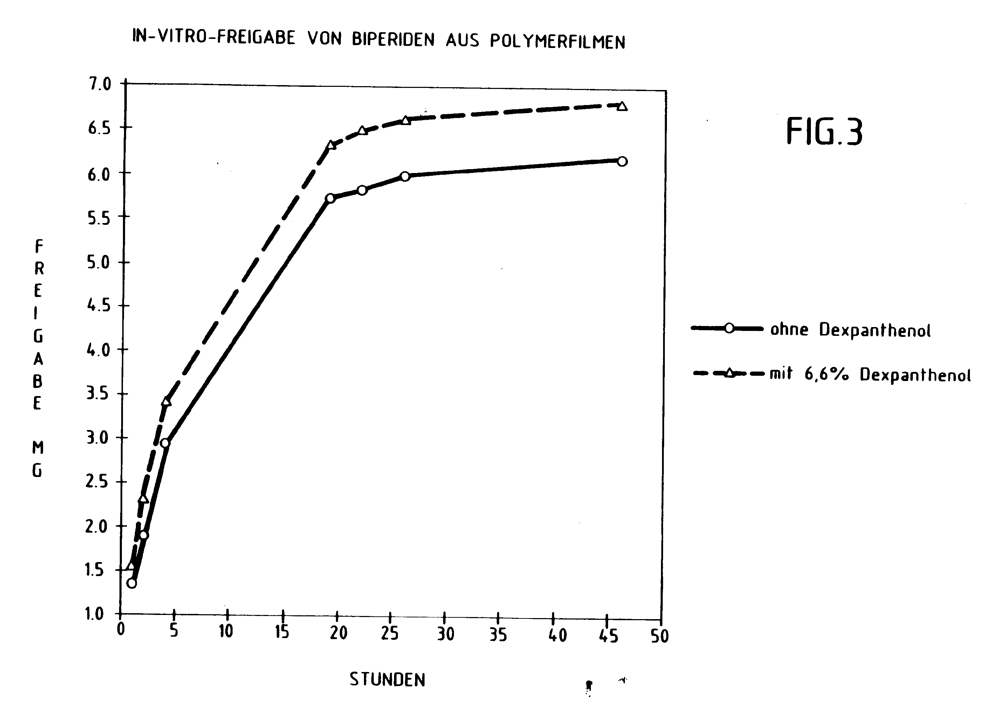

Fig. 3 zeigt, daß Dexpanthenol die Freisetzung von Biperiden beschleunigt. Dadurch kann der Haut initial mehr Biperiden angeboten werden, wodurch sich die Anflutungszeit verkürzt. Ferner bewirkt Dexpanthenol, daß, bezogen auf den Gehalt des Pflasters an Biperiden, im Verlauf eines Applikationszeitraums mehr Wirkstoff freigesetzt wird, weniger in der Matrix verbleibt und dadurch das Wirkstoffpflaster effizienter genutzt wird. Somit kann durch den Zusatz von Dexpanthenol die Beladung des Pflasters mit Wirkstoff verringert werden, ohne daß sich die Freisetzungskurve merklich ändert.

Verschiedene Gallopamilzubereitungen (Lösung in Isopropylmyristat/Ethanol 7:3, Matrixpflaster aus Polybutylacrylat, Matrixpflaster aus Polyethylhexylacrylat) mit einem Gehalt von 15 % Gallopamil wurden mit und ohne 15 % Dexpanthenol hergestellt. Alle Formulierungen wurden bei 6 Probanden auf das obere Drittel des Rückens geklebt. Für die Applikation der Lösungen wurden Epicutan-Testpflaster (Leuko-Test, Fa. Beiersdorf) verwendet. Die Fläche der Matrixpflaster betrug 2 cm². Nach 48-stündiger Applikation wurden die Pflaster entfernt und die Hautrötung nach einer 5-stufigen Skala beurteilt. Dabei war die Hautrötung unter den dexpanthenolhaltigen Pflastern signifikant geringer als unter den dexpanthenolfreien.

# EP 0 380 989 B1

|  | ohne Dexpanthenol | | mit 15 % Dexpanthenol | |
|---|---|---|---|---|
| Isopropylmyristat/Ethanol | 2 | 0 | 1 | 0 |
| | 2 | 1 | 1 | 1 |
| | 2 | 3 | 1 | 2 |
| | Summe: 10 | | Summe: 6 | |
| Polybutylacrylat | 2 | 1 | 1 | 1 |
| | 1 | 1 | 1 | 0 |
| | 1 | 2 | 1 | 1 |
| | Summe: 8 | | 5 | |
| Polyethylhexylacrylat | 1 | 1 | 0 | 0 |
| | 2 | 0 | 1 | 1 |
| | 1 | 1 | 1 | 1 |
| | Summe: 6 | | Summe: 4 | |

Bewertungsskala:

0 ≙ keine Rötung

1 ≙ sehr leichte Rötung

2 ≙ leichte Rötung

3 ≙ mittelstarke Rötung

4 ≙ starke Rötung

## Patentansprüche

1. Pflaster zur transdermalen Anwendung mindestens eines systemisch wirksamen pharmazeutischen Wirkstoffs mit einem Molekulargewicht unter 1000, dadurch gekennzeichnet, daß es zusätzlich zum Wirkstoff und üblichen galenischen Hilfsmitteln Dexpanthenol enthält.

2. Pflaster nach Anspruch 1, dadurch gekennzeichnet, daß ein Wirkstoff aus folgenden Gruppen eingesetzt wird: Opiate, Calcium-Antagonisten, Antihypertensiva, Antiarrhythmika, Beta-Rezeptorenblocker, Psychopharmaka, Vasodilatatoren, Antiparkinson-Mittel, Anticholinergika, Antihistaminika, Antirheumatika und Hormone.

3. Pflaster nach Anspruch 1, dadurch gekennzeichnet, daß der Wirkstoff Hydromorphon, Biperiden, Gallopamil oder Soquinolol ist.

4. Verwendung von Dexpanthenol als Penetrationshilfsmittel bei der Herstellung von Pflastern zur transdermalen Anwendung von systemisch wirksamen Pharmaka mit einem Molekulargewicht unter 1000.

## Claims

1. A plaster for the transdermal administration of at least one pharmaceutical active substance which has systemic activity and a molecular weight below 1000, which contains dexpanthenol in addition to the active substance and conventional pharmaceutical aids.

2. A plaster as claimed in claim 1, wherein the active substance is from the following groups: opiates, calcium antagonists, antihypertensives, antiarrhythmics, beta-receptor blockers, psychopharmaceuticals, vasodilators, antiparkinson agents, anticholinergics, antihistamines, antirheumatics and hormones.

3. A plaster as claimed in claim 1, wherein the active substance is hydromorphone, biperiden, gallopamil or soquinolol.

4. The use of dexpanthenol as a penetration aid in the production of plasters for the transdermal administration of drugs which have systemic activity and a molecular weight below 1000.

**Revendications**

1. Emplâtre pour utilisation transcutanée d'au moins un principe actif pharmaceutique à effet systémique d'un poids moléculaire inférieur à 1000, caractérisé par le fait qu'il contient du dexpanthénol en plus du principe actif et des auxiliaires galémiques usuels.

2. Emplâtre selon la revendication 1, caractérisé par le fait que l'on emploie un principe actif d'un des groupes suivants :

Opiats, antagonistes du calcium, antihypertensif, antiarythmiques, bêta-bloquants des récepteurs, produits psychopharmaceutiques vasodilatateurs, agent antiparkinson, anticholinergiques, antihistaminiques, antirhumatismes et les hormones.

3. Emplâtre selon la revendication 1, caractérisé par le fait que le principe actif est hydromorphone, bipéridène gallopamide ou soquinolole.

4. Utilisation de dexpanthénol comme auxiliaire de pénétration pour la préparation d'emplâtre destinés à l'utilisation transcutanée de produits pharmaceutiques à action systémique, d'un poids moléculaire inférieur à 1000.

PERMEATION VON HYDROMORPHON DURCH RATTENHAUT

FIG.1

Legend:
- Ethylcaprylat/ Ethanol/Dexpanthenol 3/1/1 (○)
- Ethylcaprylat/ Ethanol 3/1 (△)

Y-axis: HYDROMORPHON MG

X-axis: STUNDEN

EP 0 380 989 B1

PERMEATION VON GALLOPAMIL-BASE DURCH RATTENHAUT

FIG.2

Ethanol/Dexpanthanol 4/1

Ethanol

STUNDEN

EP 0 380 989 B1

IN-VITRO-FREIGABE VON BIPERIDEN AUS POLYMERFILMEN

FIG.3

ohne Dexpanthenol

mit 6,6% Dexpanthenol

STUNDEN

EP 0 380 989 B1